# EUROPEAN PATENT APPLICATION

(11) **EP 3 659 672 A1**
(43) Date of publication of application: **03.06.2020**
(21) Application number: 18306579.6
(22) Date of filing: 28.11.2018
(51) Int. Cl.: A61P 7/06, A61K 31/138, A61K 38/20, A61K 38/18, A61K 38/19, A61K 45/06, A61K 31/135, A61K 31/15, A61K 31/343, A61K 31/4525

(54) **COMBINED USE OF SELECTIVE SEROTONIN REUPTAKE INHIBITORS AND HEMATOPOIETIC GROWTH FACTORS FOR TREATING HEMATOPOIETIC DISEASES**

(71) Applicant: Institut Gustave Roussy, 94800 Villejuif (FR); ASSISTANCE PUBLIQUE - HÔPITAUX DE PARIS, 75004 Paris (FR); Imagine Institut des Maladies Génétiques Necker Enfants Malades, 75015 Paris (FR); Institut National de la Santé et de la Recherche Médicale, 75013 Paris (FR); UNIVERSITE PARIS DESCARTES, 75006 Paris (FR); CENTRE NATIONAL DE LA RECHERCHE SCIENTIFIQUE, 75016 Paris (FR)
(72) Inventor: COMAN, Tereza, 75015 PARIS (FR); COTE, Francine, 75012 PARIS (FR); HERMINE, Olivier, 75014 PARIS (FR); FOUQUET, Guillemette, 75014 PARIS (FR); ROSSIGNOL, Julien, 75010 PARIS (FR)
(74) Representative: A.P.I. Conseil

(57) **Abstract**

The invention relates to the combined use of selective serotonin reuptake inhibitors (SSRIs) and hematopoietic growth factors as a drug and particularly for treating cytopenia related to hematopoietic diseases or chemotherapy, and also to a pharmaceutical kit comprising both SSRIs and hematopoietic growth factors. This combination is more particularly used for treating patients presenting cytopenia, and patients in need of chemotherapy and more particularly to reduce length of aplasia after chemotherapy.

## Description

### FILED OF THE INVENTION

The present invention relates to the field of hematopoietic diseases involving cytopenia related to hematopoietic stem and progenitor cells disorders, or chemotherapy with or without hematopoietic stem cells transplantation. It relates to the combined use of selective serotonin reuptake inhibitors (SSRIs) and hematopoietic growth factors as a drug and particularly for treating these diseases, and also to a pharmaceutical kit comprising both SSRIs and hematopoietic growth factors. The combination is more particularly used for treating patients presenting cytopenia due to hematopoietic diseases and patients in need of chemotherapy and more particularly to reduce length of aplasia after chemotherapy.

### BACKGROUND OF THE INVENTION

Hematopoietic stem cells (HSCs) are the stem cells responsible for the production of mature blood cells in bone marrow. This process is called hematopoiesis and the only way by which all mature blood cells are produced. It must balance enormous production needs (more than 500 billion blood cells are produced every day) with the need to precisely regulate the number of each blood cell type in the circulation. The vast majority of hematopoiesis occurs in the bone marrow and is derived from a limited number of hematopoietic stem cells (HSCs) that are multipotent and capable of extensive self-renewal.

HSCs give rise to both the myeloid and lymphoid lineages of blood cells. Myeloid cells include monocytes to macrophages, neutrophils, basophils, eosinophils, erythrocytes, and megakaryocytes to platelets. Lymphoid cells include T cells, B cells, and natural killer cells. Myeloid and lymphoid lineages both are involved in dendritic cell formation. The hematopoietic tissue contains cells with long-term and short-term regeneration capacities and committed multipotent, oligopotent, and unipotent progenitors. HSCs constitute 1:10.000 of cells in myeloid tissue. HSC transplants are used for example in the treatment of cancers, preferably hematologic cancers and other immune system disorders.

It is well-known in the art, especially in hematology that stem and progenitor cells are widely used for treatments such as bone marrow or peripheral stem cell transplantation. Majority of autograft and 75% of allograft of hematopoietic stem cells (HSCs) are realized with HSCs from peripheral blood stem cells after mobilization.

However, 30% of donors are in failed state after mobilization (L. B. *et al. 1992;* L. B., Dyson, P. G. & Juttner, C. 1986). One strategy envisaged for treating these pathologies is to improve mobilization of peripheral blood stem cells from donors. Consequently, in the context of hematopoietic diseases, there is a need to identify new factors improving mobilization of peripheral blood stem cells from donors and thus, a better management of patients suffering from hematopoietic diseases, particularly hemopathy. This refers to needs of improving the output of chemotherapy, especially aplasia chemotherapy by reducing the duration of aplasia.

Previous studies mentioned that serotonin (5-hydroxytryptamine or 5-HT) plays a role in embryogenic development, regenerative properties, a role on stem and progenitor cells during development and after, and more specifically its role on hematopoiesis (Reviewed in Fouquet et al. 2018).

A large number of studies have focused on the role of serotonin as a neurotransmitter in the central nervous system, although only a small percentage of the body's serotonin (∼5%) can be found in the mature brain of mammals (V. Erspamer, 1937; V. Erspamer, B. Asero, 1952). In the gut, the enterochromaffin cells are scattered in the enteric epithelium from the stomach through the colon and produce over 95% of the body's serotonin. Since the generation of tryptophan hydroxylase (Tph1 and Tph2) knockout mice, unsuspected roles have been identified for serotonin synthesized outside the brain (Reviewed in S.N. Spohn, G.M. Mawe, 2017; P. Amireault et al. 2013; Mosienko V et al. 2015).

This murine model deficient in peripheral serotonin (Tph1-/-) is a unique experimental tool for exploring the molecular and cellular mechanisms involving serotonin's local effects through microserotonergic systems. The inventors previously showed and described the role of peripheral serotonin on stem cells as well as on hematopoietic progenitors, especially the role of serotonin in hematopoietic diseases, and whether targeting the serotonergic system could be of therapeutic value for the regulation of normal and pathological hematopoiesis (Fouquet et al. 2018). Further, it is known in the art that, treatments with selective serotonin reuptake inhibitors (SSRIs) such as fluoxetine, by blocking the action of SERT, lead to increased 5-HT extracellular concentrations and signaling (J.P. Feighner, 1999).

Furthermore, if each of the molecules has been tested alone in many diseases involving or not hematopoietic diseases, no research team has up to now suggested combining selective serotonin reuptake inhibitors (SSRIs) and hematopoietic growth factors in the context of the treatment of any of these diseases.

### SUMMARY OF THE INVENTION

In the context of the invention, the inventors surprisingly found that the combined use of selective serotonin reuptake inhibitors (SSRIs) and hematopoietic growth factors generates synergistic effects, thus making it possible to improve the treatment of patients suffering from cytopenia related to hematopoietic diseases or chemotherapy and can be used as a drug, preferably to improve cytopenia and after a chemotherapy to reduce the length of aplasia.

In a first aspect, the present invention thus relates to a combination of selective serotonin reuptake inhibitors (SSRIs) and hematopoietic growth factors for use as drug.

The invention further relates to a combination of selective serotonin reuptake inhibitors (SSRIs) and hematopoietic growth factors for use in the treatment of hematopoietic diseases. In a preferred embodiment, said combination is used for treating cytopenia. Cytopenia can occur after several hematopoietic diseases or chemotherapy. Accordingly, treatment of said diseases can involve a hematopoietic stem cell transplantation. Consequently, the combination of selective serotonin reuptake inhibitors (SSRIs) and hematopoietic growth factors are administered to a patient in need thereof, thus, patients that are previously treated with chemotherapy and in need of hematopoietic stem cell regeneration.

In another preferred embodiment, the drug is administered to patients in need thereof and particularly suffering of hematopoietic diseases and in need of improving Hematopoietic Stem Cells mobilization and/or engraftment function. Preferably, in the context of the invention Hematopoietic Stem Cells are collected in the peripheral blood after said mobilization. Further, the combination of selective serotonin reuptake inhibitors (SSRIs) and hematopoietic growth factors is administered in autograft or allograft context, preferably Hematopoietic stem cells (HSCs)-autograft or Hematopoietic stem cells (HSCs)-allograft.

In another preferred embodiment, selective serotonin reuptake inhibitors (SSRIs) is selected from the group consisting in fluoxetine, Citalopram, Sertraline, Paroxetine, Escitalopram, Fluvoxamine and hematopoietic growth factors is selected from the group consisting in Erythropoietin (EPO), Thrombopoietin (TPO), Granulocyte colony-stimulating factor (G-CSF), Granulocyte-macrophage colony-stimulating factor (GM-CSF), Platelet-derived growth factor (PDGF), Interleukin 3 (IL-3), Interleukin 6 (11-6).

In a second aspect, the invention also relates to a pharmaceutical kit comprising selective serotonin reuptake inhibitors (SSRIs) and hematopoietic growth factors, preferably for use as drug. Selective serotonin reuptake inhibitors (SSRIs) and hematopoietic growth factors can be present in the kit in a single formulation or in two distinct formulations.

The combination of the invention is particularly suited to improve Hematopoietic Stem Cells mobilization and/or engraftment function, to improve cytopenia and reduce the length of aplasia, preferably after chemotherapy. Therefore, the invention is particularly suited for treating diseases associated to hematopoietic disorders and/or for treating cytopenia.

### LEGEND OF DRAWING

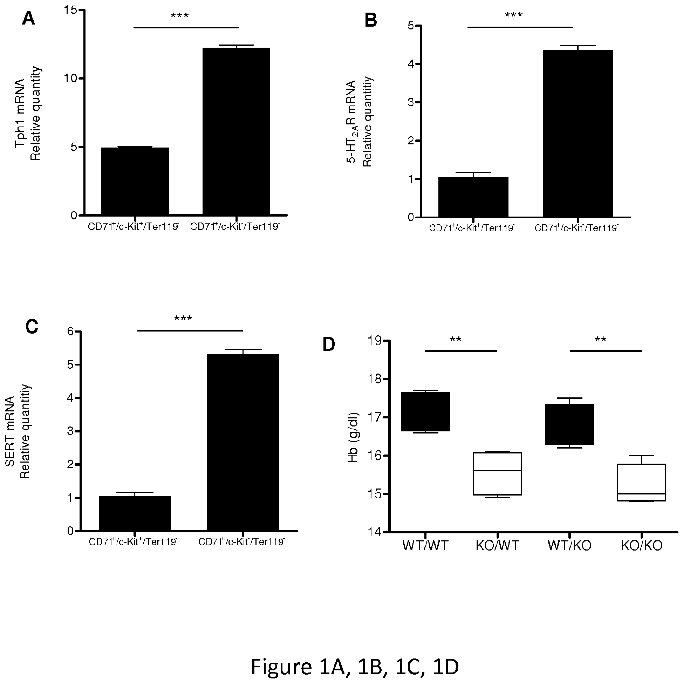

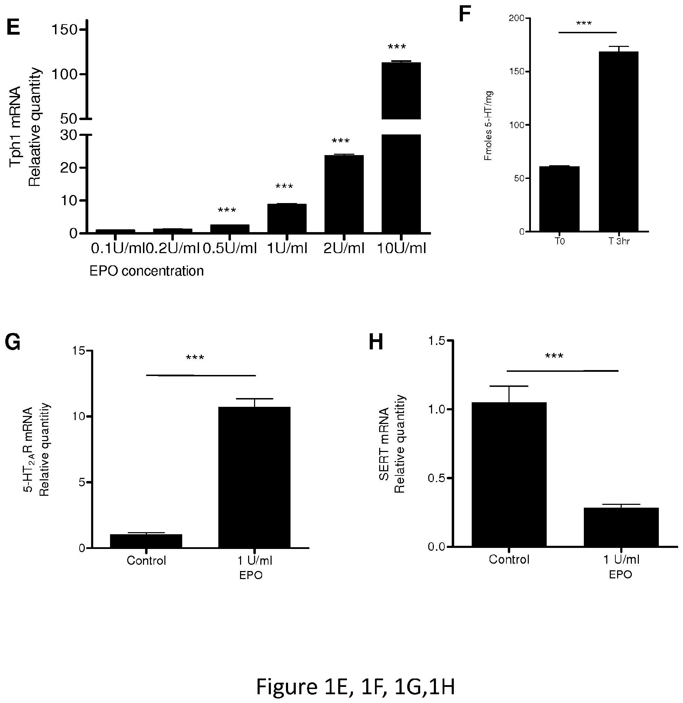

**Figure 1****. Cell-autonomous action of serotonin contributes *in vivo* to normal erythropoiesis (mouse).** Identification of components of the 5-HT system in murine progenitor cells of the bone marrow and presence of mRNA for Tph1 selectively and highly expressed at the CFU-E-to-pro-erythroblast transition checkpoint (CD71⁺/c-Kit⁺/TER119⁻ to CD71⁺/c-Kit⁻/TER119⁻) (**A**). High levels of mRNA expression encoding for SERT and 5-HT_{2A}R were also seen specifically in the pro-erythroblast population (CD71⁺/c-Kit⁻/TER119⁻) population, derived from the bone marrow of adult WT mice (2 months old) (**B-C**). 5-HT plays a cell-autonomous role as the anemic phenotype was transplantable by Tph1-/- bone marrow cells in WT mice. Specifically, WT mice that received a transplant from Tph1-/- (KO/WT) bone marrow cells had decrease Hb levels. Whereas Tph1-/- mice that received a transplant from WT bone marrow cells (WT/KO) presented no anemic phenotype (**D**). Tph1 is positively regulated by EPO in a dose-dependent manner (**E**) and 5-HT synthesis occurred as early as 3 hours following EPO stimulation (**F**). Increasing EPO concentration also up-regulated 5-HT_{2A}R but downregulated SERT (**G-H**). EPO stimulates 5-HT synthesis in proerythroblasts, up-regulates the 5-HT_{2A}R and down regulates SERT to increase extra-cellular concentrations of 5-HT and prolonged its action via the 5-HT_{2A}R.

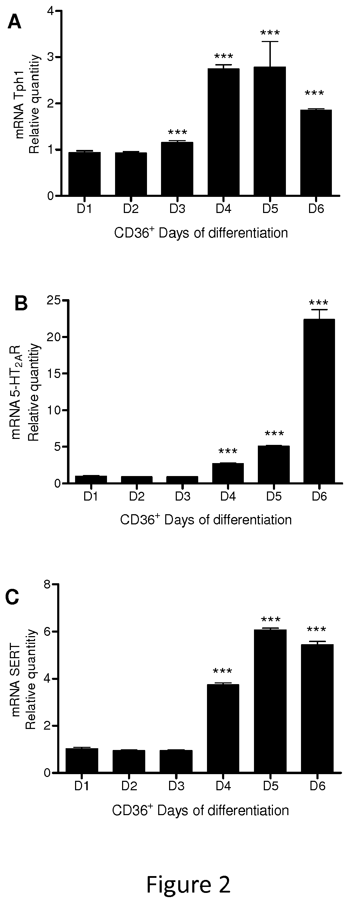

**Figure 2****. Complete serotonergic system in human erythroid progenitors.** In purified human cord blood cells, using RT-qPCR, inventors demonstrate th0at TPH1, the 5-HT2A receptor (5-HT_{2A}R-HTR2a) and the 5-HT specific membrane transporter (SERT-slc6a4) were highly expressed. In human CD36+ cord blood cells cultured with EPO; TPH1, HTR2a, and SLC6a4 genes were expressed at the pro-erythroblast stage of differentiation (day 3 of the culture after CD36+ isolation (Zermati et al., 2001) (**A-B-C**).

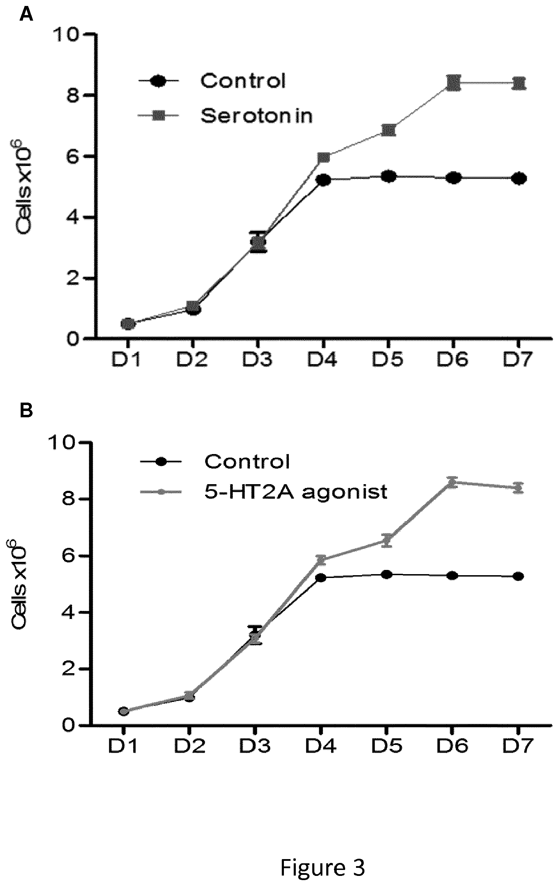

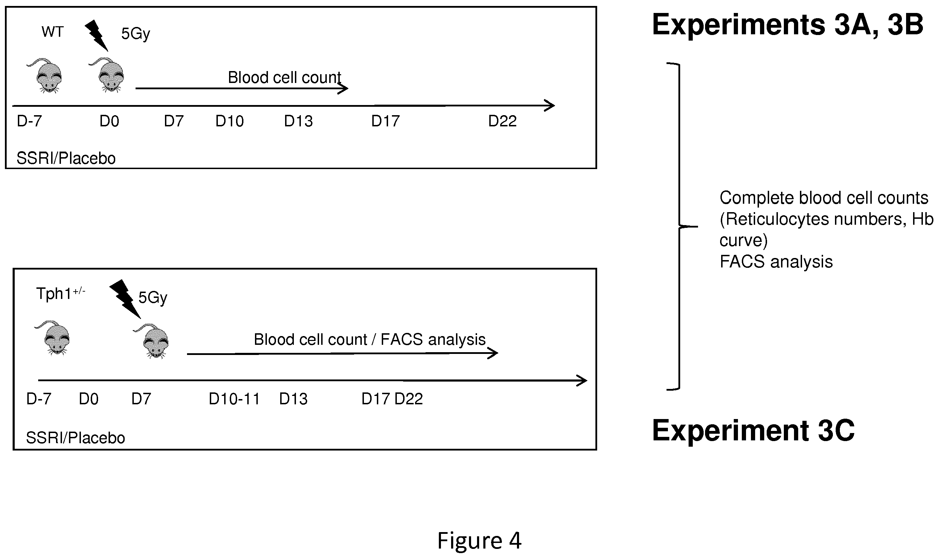

**Figure 3****. Human *in vitro* experiments (effect of 5-HT combined with EPO).** To determine the role played by serotonin in erythroid proliferation, inventors used a well-defined culture system that closely mimics the proliferation and differentiation of erythroid precursors *in vivo.* Use of 5-HT (**A**) or (**B**) PNU 22394, a 5-HT_{2A}R agonist significantly enhances erythroid proliferation. Erythroid cells were generated from CD34⁺ cord blood progenitor cells in serum-free medium in the presence of EPO (2 mU/ml) + IL-3 (10 ng/ml) + stem cell factor (SCF; 50 ng/ml). Throughout data are mean± SEM. Unpaired t-test and Pearson linear and non-linear regressions were used when appropriate. *P < 0.05, **P < 0.005, ***P < 0.0005.
**Figure 4****. Mice *in vivo* experiments (effect of 5-HT combined with EPO).** Graphic representation of the Experimental procedure for Figures 3A, 3B and 3C to understand the *in vivo* mechanism of action of 5-HT signaling on bone marrow cells was through the use of model of anemia. WT mice were sub-lethally irradiated and prior to treatment, were administered the well-known SSRI fluoxetine or a placebo.

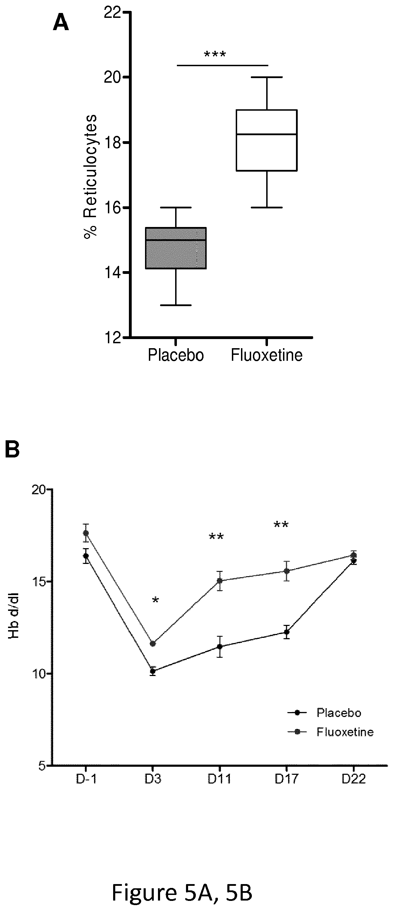

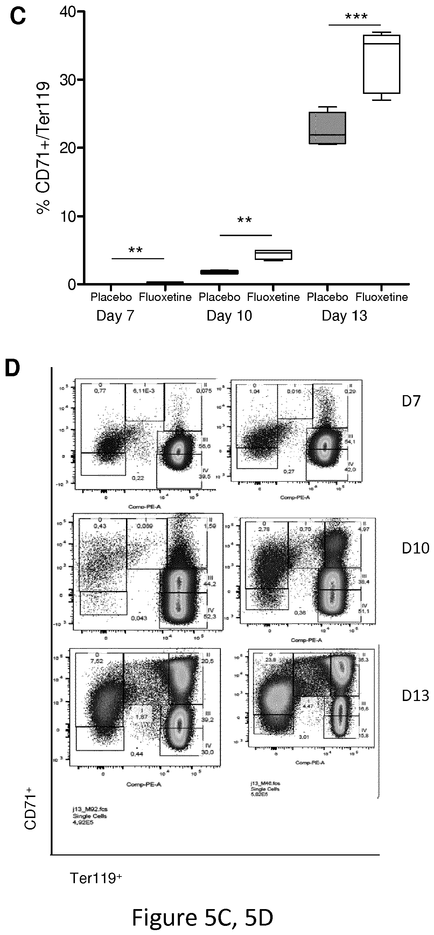

**Figure 5****. Mice *in vivo* experiments (effect of SSRI).** (**A**) % of reticulocytes and (**B**) Hb level in WT mice (n=7) treated with placebo or fluoxetine for 7 days followed by sub-lethal irradiation. (Data are from 2 independent experiments). In WT mice treated with placebo, a prolonged anemia without normalization of Hb until day 22 was observed, whereas, in WT mice treated with fluoxetine, a rapid and significant increase in the number of reticulocytes was observed as early as day 3 and a normalization of Hb levels were seen as early as day 11 (**A-B**).
   To mimic a physiological situation and relate our findings to human health issues, as we hypothesize that individuals with lower 5-HT level may be more at risk to develop myelodysplastic syndromes related anemia, inventors performed the same experiment with the heterozygous Tph1+/- mice where 5-HT level are 50% of the WT control level. Starting on day 7 following sub-lethal irradiation, inventors observed, an increase in the CD71+/TER119+ population in Tph1+/- mice treated with fluoxetine as compared to the one treated with placebo. Measurement of 5-HT levels in bone marrow revealed an increased on day 7 of treatment which correlates with the initiation of proliferation.
   The data demonstrate that pharmacological restoration of 5-HT levels through SSRI treatment prolongs and increases 5-HT_{2A}R stimulation, which in turn enhances cellular division of erythroid progenitors to rescue the proliferation defect. (**C and D**). Throughout data are mean± SEM. Paired and an unpaired t-tests were used when appropriate. *P < 0.05, **P < 0.005, ***P < 0.0005.

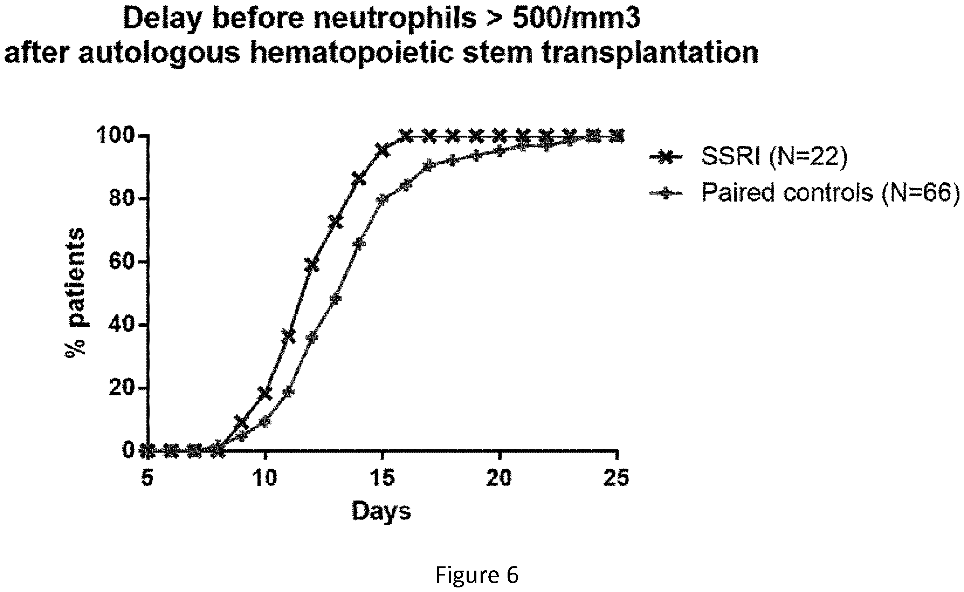

**Figure 6****. Human cohort study (effect of SSRI).** In order to evaluate the impact of treatment with SSRI on post-chemotherapy aplasia, inventors screened all patients benefiting from autologous hematopoietic stem cell transplantation (HSCT) from 2010 to 2017 in the hematology department of Necker Hospital, Paris, France. Inventors retrospectively reviewed 22 patients who were under therapy with SSRI for usual indications at the time of autologous HSCT and compared them with 66 control patients (3 controls per patient) matched for confonding factors (age, sex, disease, response of disease before therapy, conditioning regimen, date of transplant and number of CD34+ cells injected). Inventors mainly compared the duration of aplasia, as defined by the period during which the number of polynuclear neutrophils is below 500/mm3. Inventors observed a reduced duration of aplasia in patients treated with SSRI at the time of autologous HSCT.

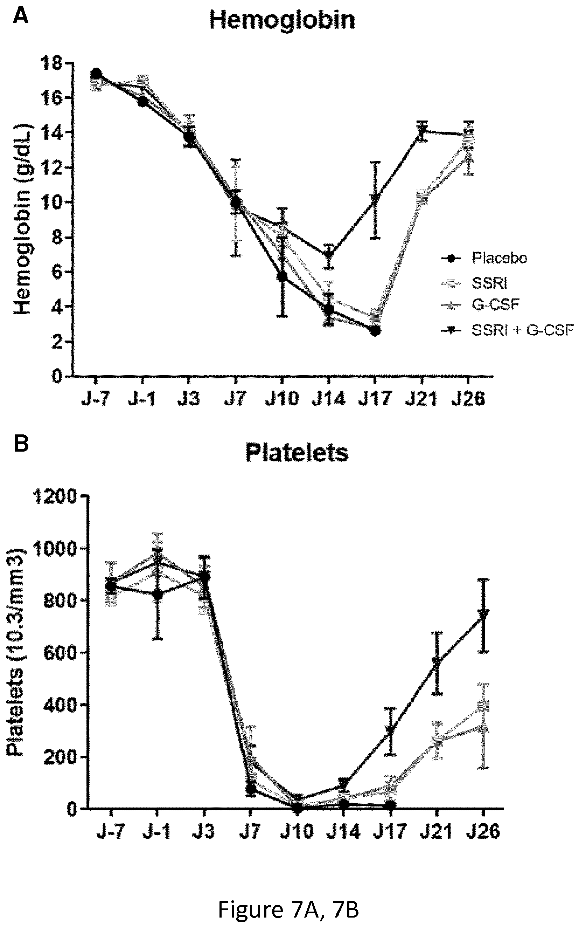

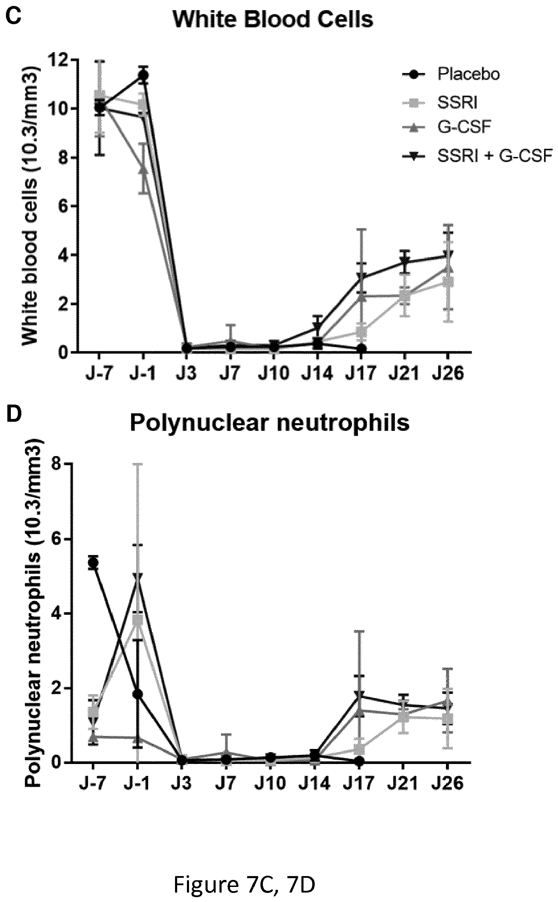

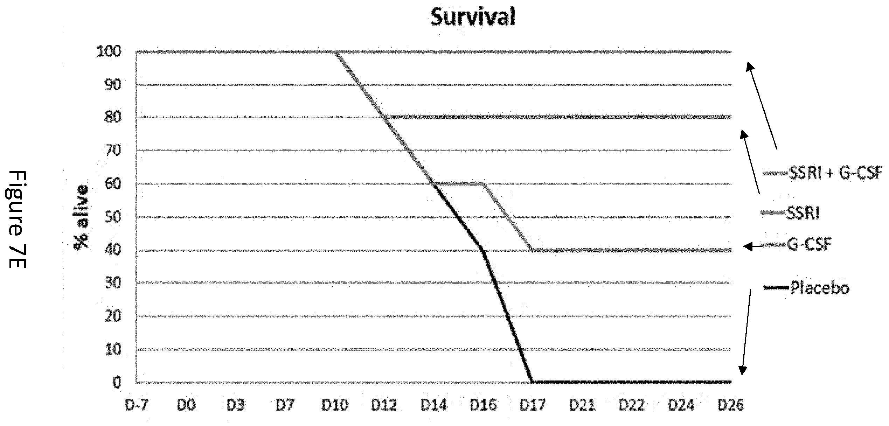

**Figure 7****. Mice *in vivo* experiments (effect of SSRI + G-CSF).** Inventors aimed to evaluate the combined effect of SSRI therapy and G-CSF on cytopenia induced by sub-lethal irradiation in mice. Inventors compared 4 groups of female 8 weeks old WT C57BL/6 mice, including 5 mice per group: placebo, SSRI (treatment with fluoxetine from 7 days before irradiation), G-CSF (treatment with G-CSF from day 4 after irradiation), SSRI + G-CSF (treatment with both fluoxetine and G-CSF as described). Inventors observed a potent effect on all myeloid lineages with a rapid increase in hemoglobin (**A**), platelets (**B**), white blood cells and polynuclear neutrophils (**C and D**). Unexpectedly, inventors also observed an improved survival in mice treated with the combination of SSRI and G-CSF (100% survival) as compared with the placebo group (0% survival) (**E**).

### DETAILED DESCRIPTION OF THE INVENTION

### Definition

As intended herein, the term "comprising" has the meaning of "including" or "containing", which means that when an object "comprises" one or several elements, other elements than those mentioned may also be included in the object. In contrast, when an object is said to "consist of" one or several elements, the object cannot include other elements than those mentioned.

According to the invention, the terms "subject", "host", "individual", and "patient" are used interchangeably herein and refer to a mammal affected or likely to be affected with disease associated with hematopoietic diseases and/or cytopenia, being more precisely a mammal presenting cytopenia or in need of chemotherapy, preferably mammal treated with chemotherapy followed by a hematopoietic stem cell transplantation. For example, a mammal suffering from hemopathy. Subjects are preferably a human being, male or female at any age that is in need of a therapy.

The term "hematopoietic diseases" or hematopoietic disorders refers to diseases that are specifically binds to Hematopoietic System. Preferably, "hematopoiesis" refers to the formation of blood cellular components. All cellular blood components are derived from hematopoietic stem cells (HSCs). In a healthy adult person, approximately 10¹¹-10¹² new blood cells are produced daily in order to maintain steady state levels in the peripheral circulation. For example, and without limiting the scope, hematopoietic diseases are hematopoietic diseases responsible for development of anemia of central or peripheral mechanism, hematopoietic diseases responsible for development of thrombopenia of central or peripheral mechanism, hematopoietic diseases responsible for development of neutropenia of central or peripheral mechanism. Preferably, hematopoietic diseases responsible for development of anemia are malignant hemopathies, myelodysplastic syndromes, hemolytic anemia, hemoglobinopathies, or aplastic anemia; hematopoietic diseases responsible for development of thrombopenia are malignant hemopathies, myelodysplastic syndromes, inherited or acquired peripheral thrombopenia, or aplastic anemia ; and hematopoietic diseases responsible for development of neutropenia are malignant hemopathies, myelodysplastic syndromes, inherited or acquired neutropenia, or aplastic anemia.

Hematopoietic stem cells (HSCs) are in the medulla of the bone (bone marrow) and have the unique ability to give rise to all of the different mature blood cell types and tissues. HSCs are self-renewing cells: when they differentiate, about 50% of their daughter cells remain as HSCs, so stem cells are not depleted. The other 50% of daughters of HSCs (myeloid and lymphoid progenitor cells) can follow any of the other differentiation pathways that lead to the production of one or more specific types of blood cell. All blood cells are divided into two lineages: myeloid and lymphoid.
- Cells of the myeloid lineage, which include erythroblasts and red blood cells, also called erythrocytes, megakaryocytes and platelets, granulocytes, macrophages and monocytes, are derived from common myeloid progenitors, and are involved in such diverse roles as oxygen transport, innate immunity and blood clotting. Erythrocytes are functional and are released into the blood. The number of reticulocytes, immature red blood cells, gives an estimate of the rate of erythropoiesis This is myelopoiesis.
- Lymphocytes derived from common lymphoid progenitors. The lymphoid lineage is composed of T-cells, B-cells and natural killer cells. This is lymphopoiesis.

As used herein, the term "Hematopoietic stem cell transplantation" or "HSCT" is the transplantation of hematopoietic stem cells, usually derived from bone marrow, peripheral blood, or umbilical cord blood. It may be autologous (the patient's own stem cells are used), allogeneic (the stem cells come from a non-identical donor of the same species) or syngeneic (from an identical twin). It is most often performed for patients with certain cancers of the blood (hemopathy) or bone marrow, such as multiple myeloma or leukemia. In these cases, the recipient's immune system is usually destroyed with radiation or chemotherapy before the transplantation. Infection and graft-versus-host disease are major complications of allogeneic HSCT.

The term "chemotherapy" refers to a treatment that uses drugs or radiation, preferably to stop the growth of cancer cells, either by killing the cells or by stopping them from dividing. Chemotherapy may be given by mouth, injection, or infusion, or on the skin, depending on the used. It may be given alone or with other treatments, such as surgery, radiation therapy, or biologic therapy. Alternately, treatment is given to patient in need of HSCT.

The term "Graft- Versus-Host Disease (GVHD)" refers to a common and serious complication wherein there is a reaction of donated immunologically competent lymphocytes against a transplant recipient's own tissue. GVHD is a possible complication of any transplant that uses or contains stem cells from either a related or an unrelated donor.

Consequently, Hematopoietic stem cell transplantation remains a dangerous procedure with many possible complications; it is reserved for patients with life-threatening diseases. As survival following the procedure has increased, its use has expanded beyond cancer to autoimmune diseases and hereditary skeletal dysplasias; notably malignant infantile osteopetrosis and mucopolysaccharidosis.

The term "cancer" as used herein is defined as disease characterized by the rapid and uncontrolled growth of aberrant cells. Cancer cells can spread locally or through the bloodstream and lymphatic system to other parts of the body. In the context of the invention cancer is preferably hematopoietic malignancy. Hematopoietic malignancy refers to tumors that affect the blood, bone marrow, lymph, and lymphatic system including lymphoid organs. All those elements are all intimately connected through both the circulatory system and the immune system, a disease affecting one will often affect the others as well, making myeloproliferation and lymphoproliferation, and thus the leukemias and the lymphomas.

Hematopoietic malignancies derive from either of the two major blood cell lineages: myeloid and lymphoid cell lines. Lymphomas, lymphocytic leukemias, and myeloma are from the lymphoid line, while acute and chronic myelogenous leukemia, myelodysplastic syndromes and myeloproliferative diseases are myeloid in origin.

"Myelodysplastic syndrome" or "MDS" refers to a group of cancers in which immature blood cells in the bone marrow do not mature and therefore do not become healthy blood cells. Symptoms may include feeling tired, shortness of breath, easy bleeding, or frequent infections. Risk factors include previous chemotherapy or radiation therapy, exposure to certain chemicals such as tobacco smoke, pesticides, and benzene, and exposure to heavy metals such as mercury or lead. In about 1 in 3 patients, MDS can progress to a cancer of bone marrow cells called acute myeloid leukemia (AML). Because all patients do not get leukemia, MDS used to be classified as a disease of low malignant potential. But now that we have learned more, MDS is considered to be a form of cancer. Treatments may include supportive care, drug therapy, and stem cell transplantation.

In the case of stem cell transplantation and particularly hematopoietic stem cell transplantation, there is a previous step of mobilization of HSCs. Hematopoietic stem cells (HSCs) normally reside in the bone marrow but can be forced into the blood. "Mobilization of HSCs" refers to this process which are used clinically to harvest large numbers of HSCs for transplantation. Currently, the mobilizing agent of choice is granulocyte colony-stimulating factor. However, not all patients mobilize well.

In the context of the invention, "Engraftment function" occurs after autologous HSCT within 7-14 days and from 14 to 28 days after allogeneic HSCT and refers to the step of engraftment of HSCs in the bone marrow niches to reconstitute immunity. Under optimal circumstances, the recipient's immune system tolerates donor cell engraftment without non-engraftment or late graft failure. Further, improvements in engraftment kinetics reduce transplantation costs.

"Treating" or "treatment of a disease or condition" refers to any act intended to ameliorate the health status of patients. "Treatment" can include, but is not limited to, alleviation or amelioration of one or more symptoms or conditions, diminishment of extent of disease, stabilization of the state of disease (e.g. maintaining a patient in remission), prevention of the disease or prevention of the spread of disease, delay or slowing of disease progression, amelioration or palliation of the disease state, diminishment of the reoccurrence of disease, and remission (whether partial or total). A treatment may include curative, alleviation or prophylactic effects. The term "prophylactic" may be considered as reducing the severity or the onset of a particular condition. "Prophylactic" also includes preventing reoccurrence of a particular condition in a patient previously diagnosed with the condition. "Therapeutic" may also reduce or delay the severity of an existing condition. Desirable effects of treatment include decreasing the rate of disease progression, ameliorating or palliating the disease state, and remission or improved prognosis. Alleviation can occur prior to signs or symptoms of the disease or condition appearing, as well as after their appearance. Thus, "treating" or "treatment" may include "preventing" or "prevention" of disease or undesirable condition. In one embodiment, treating a cancer includes inhibiting the growth or proliferation of cancer cells or killing cancer cells. In a particular embodiment, treating a cancer includes reducing the risk or development of metastasis. In another particular embodiment, treating a cancer may refer to the prevention of a relapse. Treating a cancer may also refer to maintaining a subject in remission.

As used herein, the terms "disorder" or "disease" refer to the incorrectly functioning organ, part, structure, or system of the body resulting from the effect of genetic or developmental errors, infection, poisons, nutritional deficiency or imbalance, toxicity, or unfavourable environmental factors. Preferably, these terms refer to a health disorder or disease e.g. an illness that disrupts normal physical or mental functions. More preferably, the term disorder refers to immune and/or inflammatory diseases that affect animals and/or humans. Preferably, the term disorder or disease refers to cancers, infectious diseases or immune diseases. The term "immune disease" or "auto-immune disease", as used herein, refers to a condition in a subject characterized by cellular, tissue and/or organ injury caused by an immunologic reaction of the subject to its own cells, tissues and/or organs.

### Treatment

The term "SSRIs" or "Selective Serotonin Reuptake Inhibitors" are a class of drugs that are typically used as antidepressants in the treatment of major depressive disorder and anxiety disorders. The exact mechanism of action of SSRIs is unknown. SSRIs are believed to increase the extracellular level of the neurotransmitter serotonin by limiting its reabsorption (reuptake) into the presynaptic cell, increasing the level of serotonin in the synaptic cleft available to bind to the postsynaptic receptor.

As used herein, the term "serotonin", "5-hydroxytryptamine" or "5-HT" refers to a monoamine neurotransmitter which is highly conserved throughout evolution. The indoleamine molecule derives from the amino acid tryptophan. Serotonin is primarily found in the enteric nervous system located in the gastrointestinal tract (Gl tract). However, it is also produced in the central nervous system (CNS). Additionally, serotonin is stored in blood platelets and is released during agitation and vasoconstriction, where it then acts as an agonist to other platelets. 5-HT is involved in cognition, attention, emotion, pain, sleep, and arousal, to name a few examples. Yet only a small percentage of the body's 5-HT (∼5%) can be found in the mature brain of mammals: most (∼95%) is found synthesized in the gastrointestinal tract. The cellular effects of 5-HT are exerted through activation of any of 15 different receptors in 7 different classes (5-HT1 to 5-HT7). Through these receptors, 5-HT can produce opposing effects that add to the complexity of the serotonergic system.

For example, SSRIs are fluoxetine, Citalopram, Sertraline, Paroxetine, Escitalopram, Fluvoxamine. By "fluoxetine", it refers to (RS)-N-méthyl-3-phényl-3--propan-1-amine (CAS no. 54910-89-3) of formula (I) below:

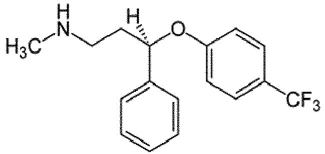

By "Citalopram", it refers to (RS)-1-[3-(diméthylamino)propyl]-1-(4-fluorophényl)-1,3-dihydro[3,4]benzofuran-5-carbonitrile (CAS no. 59729-33-8) of formula (II) below:

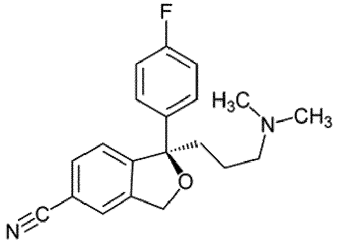

By "Sertraline", it refers to (1*S*,4*S*)-4-(3,4-dichlorophényl)-N-méthyl-1,2,3,4-tétrahydronaphtalén-1-amine (CAS no. 79617-96-2) of formula (III) below:

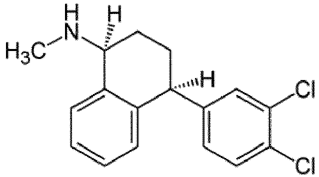

By "Paroxetine", it refers to (3S,4P)-3-[(1,3-benzodioxol-5-yloxy)méthyl]-4-(4-fluorophényl)pipéridine (CAS no. 61869-08-7) of formula (IV) below:

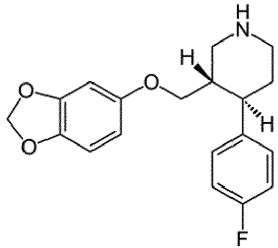

By "Escitalopram", it refers to (1S)-1-[3-(diméthylamino)propyl]-1-(4-fluorophényl)-3H-2-benzofuran-5-carbonitrile (CAS no. 128196-01-0) of formula (V) below:

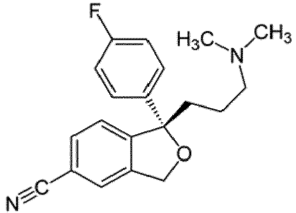

By "Fluvoxamine", it refers to (*E*)-2-[(5-méthoxy-1-[4-(trifluorométhyl)phényl] pentylidène)amino]oxyéthanamine (CAS no. 54739-18-3) of formula (VI) below:

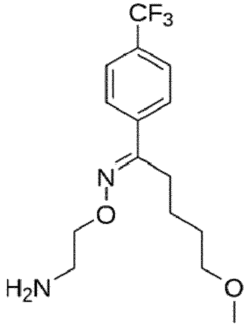

The term "hematopoietic growth factors" refers to factors who regulate the differentiation and the proliferation of particular progenitor cells. Granulocyte-macrophage colony-stimulating factor and granulocyte CSF are given to stimulate white blood cell formation in cancer patients who are receiving chemotherapy, which tends to kill their bone marrow cells as well as the cancer cells. Thrombopoietin shows great promise for preventing platelet depletion during chemotherapy. CSFs and thrombopoietin also improve the outcome of patients who receive bone marrow transplants. For example, hematopoietic growth factors are Erythropoietin (EPO), Thrombopoietin (TPO), Granulocyte colony-stimulating factor (G-CSF), Granulocyte-macrophage colony-stimulating factor (GM-CSF), Platelet-derived growth factor (PDGF), Interleukin 3 (IL-3), Interleukin 6 (11-6). Erythropoietin or EPO is a glycoprotein cytokine secreted by the kidney in response to cellular hypoxia; it stimulates red blood cell production (erythropoiesis) in the bone marrow. Thrombopoietin (TPO), also known as megakaryocyte growth and development factor (MGDF), refers to a protein that in humans is encoded by the TPO gene. Particularly, it is a glycoprotein hormone produced by the liver and kidney which regulates the production of platelets. It stimulates the production and differentiation of megakaryocytes, the bone marrow cells that bud off large numbers of platelets. Granulocyte-colony stimulating factor (G-CSF or GCSF), also known as colony-stimulating factor 3 (CSF 3), is a glycoprotein that stimulates the bone marrow to produce granulocytes and stem cells and release them into the bloodstream. Granulocyte-macrophage colony-stimulating factor (GM-CSF), also known as colony-stimulating factor 2 (CSF2), is a monomeric glycoprotein secreted by macrophages, T cells, mast cells, natural killer cells, endothelial cells and fibroblasts that functions as a cytokine. Unlike granulocyte colony-stimulating factor, which specifically promotes neutrophil proliferation and maturation, GM-CSF affects more cell types, especially macrophages and eosinophils. Platelet-derived growth factor (PDGF) is one of numerous growth factors that regulates cell growth and division. In particular, PDGF plays a significant role in blood vessel formation. Interleukin 3 (IL-3) is a T cell-derived pluripotent hematopoietic colony-stimulating factor required for the survival and proliferation of primitive hematopoietic progenitor cells. Interleukin 6 (IL-6) is an interleukin that acts as both a pro-inflammatory cytokine and an anti-inflammatory myokine.

Despite the teachings of the prior art, according to which selective serotonin reuptake inhibitors (SSRIs) are typically used as antidepressants in the treatment of major depressive disorder, the inventors surprisingly found that the combined use of selective serotonin reuptake inhibitors (SSRIs) and hematopoietic growth factors generates synergistic effects, thus making it possible to obtain better treatment of cytopenia, related to hematopoietic diseases and especially after a chemotherapy. In a particular aspect, said combination generates synergistic effects and reduce the duration of aplasia after chemotherapy.

This synergy was shown in *in vivo* experiments after orally administration of fluoxetine combined with EPO and in human cohort. The experiments show that the length of aplasia after chemotherapy or irradiation is considerably reduced.

Accordingly, the present invention relates to the use of a combination of selective serotonin reuptake inhibitors (SSRIs) and hematopoietic growth factors as drug. In a particular embodiment of the invention, said combination is particularly suited after chemotherapy, especially to improve and reduce aplasia length after chemotherapy. By "Aplasia", it refers to cytopenia occurring on all hematopoietic lineages. After chemotherapy, aplasia relates to a temporary defective production and/or maturation of cells of all hematopoietic lineages. It represents the length that there is a defective development of hematopoietic stem or progenitor cells. Especially, after chemotherapy, there is a temporary blockage of the activity of bone marrow or hematopoiesis inducing a decrease of the production of blood cells. This period of time is particularly critical because the patients are more vulnerable and so, there is a high risk of infection.

In another embodiment of the invention, said combination is suited for a use in the treatment of hematopoietic diseases, especially for patient in need of hematopoietic stem cell transplantation (HSCT).

Previous HSCT, conditioning treatment is administered to patient in need thereof to eliminate the underlying disease, create space for the new marrow and prevent rejection of the new bone marrow. Once the conditioning treatment has begun, patients usually need to be in protective isolation to help prevent infection. Protective isolation means that it is necessary for the patient to remain in the hospital room or ward most of the time. Protective isolation continues throughout transplant and for about three weeks post-transplant, until the patient's condition and white blood cell count have improved to a satisfactory level. There is a variety of conditioning regimens that involve chemotherapy alone, or a combination of chemotherapy and total body irradiation (TBI).

Chemotherapy. All patients will receive chemotherapy drugs prior to the blood and marrow transplant. The chemotherapy is given in high doses in order to eliminate the disease or cancer. In the case of an allogeneic (donor), chemotherapy suppresses the immune system to allow the transplanted bone marrow to undergo a process called engraftment.

Total Body Irradiation (TBI). Some patients will receive radiation therapy in addition to chemotherapy during their conditioning treatment. Like chemotherapy, total body irradiation (TBI) is used to eliminate the disease and in the case of donor or allogeneic transplant, to suppress the patient's immune system in preparation for the transplanted stem cells.

In the context of the present invention, hematopoietic diseases comprise:
- hematopoietic diseases responsible for development of anemia of central or peripheral mechanism,
- hematopoietic diseases responsible for development of thrombopenia of central or peripheral mechanism,
- hematopoietic diseases responsible for development of neutropenia of central or peripheral mechanism.

In a particular embodiment, hematopoietic diseases responsible for development of anemia comprise malignant hemopathies, myelodysplastic syndromes, hemolytic anemia, hemoglobinopathies, or aplastic anemia.

In another particular embodiment hematopoietic diseases responsible for development of thrombopenia comprise malignant hemopathies, myelodysplastic syndromes, inherited or acquired peripheral thrombopenia, or aplastic anemia.

In another particular embodiment hematopoietic diseases responsible for development of neutropenia comprise malignant hemopathies, myelodysplastic syndromes, inherited or acquired neutropenia, or aplastic anemia.

In another particular embodiment, said combination is administered to a patient for the treatment of cytopenia. By "cytopenia", it refers to context when one or more of patient blood cell types is lower than it should be. Blood consists of three main parts. Red blood cells, also called erythrocytes, carry oxygen and nutrients around your body. White blood cells, or leukocytes, fight infection and battle unhealthy bacteria. Platelets are essential for clotting. If any of these elements are below typical levels, patients have cytopenia. Several types of cytopenia exist:
- Anemia occurs when your red blood cells are low.
- Leukopenia is a low level of white blood cells.
- Thrombocytopenia is a deficiency of platelets.
- Pancytopenia is a deficiency of all three myeloid lineages in the blood.

But, the possible causes of cytopenia are complex and varied. Among these causes are peripheral destruction, infections, and side effects of medication. Two types of cytopenia that are related to the underlying cause of the low blood cell count are autoimmune cytopenia and refractory cytopenia. Autoimmune cytopenia is caused by an autoimmune disease. Your body produces antibodies that fight against your healthy blood cells, destroying them and preventing you from having adequate blood cell counts. Refractory cytopenia occurs when your bone marrow does not produce mature, healthy blood cells. This may be the result of a group of cancers, such as leukemia or another bone marrow condition.

In a particular embodiment of the invention, combination of selective serotonin reuptake inhibitors (SSRIs) and hematopoietic growth factors improve hematopoietic stem and progenitor cells regeneration. Hence, this combination is preferably for use as a drug wherein diseases involve or require hematopoietic stem and progenitor cells regeneration. Accordingly, said combination reduce the duration of aplasia and so, the risk of infection for patient having received a hematopoietic stem cell transplantation. The reduction of the duration of aplasia can be related to a direct or indirect effect of the combination of SSRIs and hematopoietic growth factors on hematopoietic stem and progenitor cells, to improve their survival, proliferation and/or maturation. This can relate to the field of regenerative medicine, which, in hematology, is particularly interesting in the context of stem cell transplantation.

In another preferred embodiment, the combination of selective serotonin reuptake inhibitors (SSRIs) and hematopoietic growth factors comprising a selective serotonin reuptake inhibitors (SSRIs) which is selected from the group consisting in fluoxetine, Citalopram, Sertraline, Paroxetine, Escitalopram, Fluvoxamine.

In another preferred embodiment, the combination of selective serotonin reuptake inhibitors (SSRIs) and hematopoietic growth factors comprising hematopoietic growth factors which is selected from the group consisting in Erythropoietin, Thrombopoietin, G-CSF, IL-3, 11-6, GM-CSF, G-CSF, PDGF.

In a particular embodiment, the combination of the present invention is particularly suited to improve hematopoietic stem and progenitor cells regeneration. Accordingly, inventors shown that said combination reduce the duration of aplasia and so, improve Hematopoietic Stem Cells mobilization and/or engraftment function. Therefore, said combination is particularly of interest for patient with cytopenia disorders. Preferably, said combination are administered to patient suffering of cytopenia secondary to hematopoietic diseases and/or chemotherapy.

In a preferred embodiment of the present invention, said combination is used to treat hematopoietic disease in allograft or autograft context. In particular, said autograft is a hematopoietic stem cells (HSCs)-autograft. In another object, said allograft is a hematopoietic stem cells (HSCs)-allograft. Thus, the combination is administered when aplasia chemotherapy is used before autologous transplant, especially autologous hematopoietic stem cells (HSCs) transplant.

In an embodiment of the invention, said combination is particularly of interest for cancer characterized in that it is hematopoietic malignancies. In a preferred embodiment, the present invention relates to a combination of selective serotonin reuptake inhibitors (SSRIs) and hematopoietic growth for treating myelodysplastic syndrome, inherited or acquired neutropenia, or aplastic anemia. Accordingly, the present invention is suited to treat myelodysplastic syndrome selected in the group consisting in refractory anemia (RA), refractory anemia with ring sideroblasts (RARS), refractory anemia with excess blasts (RAEB), refractory anemia with excess blasts in transformation (RAEB-T), chronic myelomonocytic leukemia (CMML).

In the context of the myelodysplastic syndrome, the combination according to the invention is preferably selective serotonin reuptake inhibitors (SSRIs) and interleukin-3 receptor (IL-3). Alternately, said combination is serotonin reuptake inhibitors (SSRIs) and interleukin-6 receptor (IL-6). Alternately, said combination is serotonin reuptake inhibitors (SSRIs) and Granulocyte-macrophage colony-stimulating factor (GM-CSF).

In another embodiment, said combination is particularly suited to treat ineffective erythropoiesis, such as the promotion of erythropoiesis, the correction of ineffective erythropoiesis. Further, said combination is preferably used for treating anemia, such as a correction of anemia, in particular during depression or the correction of anemia in myelodysplastic syndromes. In the context of erythropoiesis disorders, the combination according to the invention is preferably selective serotonin reuptake inhibitors (SSRIs) and Erythropoietin (EPO).

In another embodiment, said combination is particularly suited to treat hemostasis disorders and/or thrombosis disorders. Accordingly, the combination is preferably selective serotonin reuptake inhibitors (SSRIs) and Platelet-derived growth factor (PDGF).

In another embodiment, said combination is particularly suited to treat auto-immune disorders and/or inflammation disorders. Accordingly, the combination is preferably serotonin reuptake inhibitors (SSRIs) and Granulocyte-colony stimulating factor (G-CSF).

### Administration

The administrations of selective serotonin reuptake inhibitors (SSRIs) and hematopoietic growth factors can be simultaneous or sequential, these terms being defined above.

Whether the administration is simultaneous or sequential, the selective serotonin reuptake inhibitors (SSRIs) and hematopoietic growth factors, can be administered by any appropriate route of administration, in particular by oral route. In a preferred embodiment, the selective serotonin reuptake inhibitors (SSRIs) and hematopoietic growth factors, are both administered by oral route, since it is the recommended route of administration for each of these two active ingredients.

Although there is to date no combined formulation of selective serotonin reuptake inhibitors (SSRIs) and hematopoietic growth factors, the development of such a formulation would make it possible to simplify the simultaneous administration of these two active ingredients for patients.

The synergistic effects shown by the inventors make it possible to reduce significantly the duration of aplasia. In the prior art, the doses of fluoxetine administered to patients suffering from mood disorders and especially depressive disorders were 20 mg/kg/day orally. Futhermore, the dose and administration mode are thus known for every drug previously mentioned, such as Fluoxetine, Citalopram, Sertraline, Paroxetine, Escitalopram, Fluvoxamine.

In another aspect, hematopoietic growth factors are well known in the art and are frequently used in cytopenia resulting from various hematopoietic and non-hematopoietic disorders or chemotherapy. For example, and without limiting the scope, EPO is used in anemia of hematopoietic origin as follows: darbopoetine α is administered in 150 to 300 µg/week or 500 µg/3 weeks, epoietine β is administered in 30 000 Ul/week; epoietine α is administered in 40 000 Ul/week.

For example, TPO agonists are used in thrombopenia of hematopoietic origin as follows: eltrombopag is administered in 25 to 75 mg/day; romiplostim is administered in 250 to 500 µg/week and maximum 10 µg/kg/week.

For example, G-CSF are used of in neutropenia hematopoietic origin as follows: lenograstim is administered in 13 or 34, 1 injection/day; filgrastim is administered in 30 or 48, 1 injection/day; tevagrastim is administered in 30 or 48, 1 injection/day; pegfilgrastim is administered in 6 mg once.

### Pharmaceutical composition

In a second aspect, the invention also relates to a pharmaceutical kit comprising selective serotonin reuptake inhibitors (SSRIs) and hematopoietic growth factors.

In the pharmaceutical kit according to the invention, selective serotonin reuptake inhibitors (SSRIs) and hematopoietic growth factors can be present in the kit in a single formulation associating the two active ingredients 1) selective serotonin reuptake inhibitors (SSRIs) and 2) hematopoietic growth factors, or of two distinct formulations each comprising one of the active ingredients (thus allowing simultaneous or sequential administration)

Further aspects and advantages of the invention will be disclosed in the following examples, which should be considered illustrative.

### EXAMPLE

### Materials and Methods.

### Animal procedures

Tph1^{-/-} mice were generated as described (Côté et al., 2003). Tph1^{-/-} and WT animals were derived from pure C57BL/6J genetic backgrounds. For some experiments, C57/bl6 mice were purchased from Janvier Labs. Animal experiments were performed according to the recommendations of the French Institutional Committee.

### Blood counts

To perform complete blood counts, peripheral blood from the tail vein was collected in EDTA tubes and analyzed using an electronic hematology particle counter ((IDEXX Procyte).

### Fluoxetine treatment

Fluoxetine was administrated orally (40 mM in water bottle (*Prozac* ® 20mg/5mL solution) to WT mice (males 8-10 weeks old C57/bl6 purchased form Janvier labs) for 7 days before inducing anemia.

### G-CSF treatment

G-CSF treatment was administered subcutaneously daily from day 4 after irradiation and until resolution of aplasia (day 17 in Figure 5), at the dose of 200 µg/kg/day.

### Sub-lethal irradiation

Sub-lethal irradiation was induced by submitting WT mice to 1.09gy during 4 minutes (WT C57/bl6 Males 8-10 weeks old purchased form Janvier labs). Sub-lethal irradiation was induced by submitting Tph1^{+/-} mice to 1.09gy during 4 minutes (Males 8-10 weeks old).

### Bone marrow transfert experiment

Bone marrow transplantation was performed as described in D'Aveni et *al.,* 2015.

### Cell culture

*Human cord blood Erythroid in Vitro Cell Culture.*

Erythroid cells were generated from CD34⁺ cord blood progenitor cells in serum-free medium in the presence of EPO (2 mU/ml) + IL-3 (10 ng/ml) + stem cell factor (SCF; 50 ng/ml) as previously described in Zermati et al., (2000) Exp. Hematol. 28:885-894.

*Immunophenotyping of Murine Erythroid Precursors.*

Cells from fetal livers or BM cells flushed from femur and tibia were resuspended in Hank's buffered saline before being passed through a 100-µM strainer. Cells were washed, counted, and immunostained at room T° in PBS with PE-conjugated anti-TER119, FITC-conjugated anti-CD71 and APC-conjugated anti-CD117 (c-Kit) (BD Biosciences) antibodies for 20 min and analyzed on a FACS Canto II coupled with FlowJo software version X.0.7 (Tree Star, Ashland, OR).

### RT qPCR

Total RNA was extracted from bone marrow cells using the RNeasy Kit (Qiagen). Reverse transcription was performed using iScript™ Reverse Transcription Supermix for RT-qPCR (BioRad). Real time PCR was performed on a STEPONE™ cycling machine (Applied Biosystems) using oligos from Taqman (Life technologies). Two biological replicates were used for each condition. Data were analyzed by StepOne Plus RT PCR software v2.1 and Microsoft excel. ß-actin, GAPDH and 18S transcript levels were used for normalisation of each target (=ΔCT). Real-time PCR CT values were analysed using the 2-(ΔΔCt) method to calculate the fold expression (ΔΔCT method).

### Patients

In order to evaluate the impact of treatment with SSRI on post-chemotherapy aplasia, inventors screened all patients benefiting from autologous hematopoietic stem cell transplantation (HSCT) from 2010 to 2017 in the hematology department of Necker Hospital, Paris, France. Inventors retrospectively reviewed 22 patients who were under therapy with SSRI for usual indications at the time of autologous HSCT, and compared them with 66 control patients (3 controls per patient) matched for confonding factors (age, sex, disease, response of disease before therapy, conditioning regimen, date of transplant and number of CD34+ cells injected). Inventors mainly compared the duration of aplasia, as defined by the period during which the number of polynuclear neutrophils is below 500/mm3. Inventors observed a reduced duration of aplasia in patients treated with SSRI at the time of autologous HSCT.

### Statistical analysis

Statistical analysis was performed using GraphPad Prism software. Throughout data are mean± SEM. Unpaired t-test and Pearson linear and non-linear regressions were used when appropriate. *P < 0.05, **P < 0.005, ***P < 0.0005.

### Results

With regard to the role played by serotonin in red blood cells production, the pharmacological and genetic evidences presented demonstrate that a functional autocrine serotonergic network exists in both murine and human erythroid progenitors. Tph1, the rate-limiting enzyme for peripheral serotonin synthesis is a novel erythroid gene. Serotonin is regulating hematopoietic stem cell fate along the erythroid pathway. The cytokine erythropoietin (EPO) induces *TPH1* expression and serotonin synthesis necessary for erythroid progenitor's survival and proliferation. Together, the data demonstrate the existence of a synergy between serotonin and EPO to stimulate human pro-erythroblast proliferation. Our *in vivo* findings imply that increasing the concentration of bone marrow serotonin available to erythroid progenitors could be a valuable therapeutic intervention for myelodysplastic anemia before leukemic onset. Thus, the use of selective serotonin reuptake inhibitors (SSRIs) such as fluoxetine, a common antidepressant may have important clinical benefit in anemia treatment in combination with EPO, especially in myelodysplastic syndromes.

With regard to the role played by serotonin in the bone marrow, it could be used as a mitogen to regulate hematopoietic stem/progenitor cells maintenance and regeneration after various types of injury. Inventors show that SSRI treatment could have an impact on the engraftment, function, and/or mobilization of HSCs. Our data demonstrate a reduction in the duration of aplasia in patients treated with SSRI at the time of autologous HSCT. Similar to the data obtained on erythroid progenitors, inventors show that SSRIs in combination with growth factors (TPO, G-CSF, PDGF, 11-3, IL-6) can be used for treating patients presenting cytopenia due to hematopoietic diseases and patients in need of chemotherapy and more particularly to reduce length of aplasia after chemotherapy.

### Conclusion

To conclude, results presented previously show that the combination of selective serotonin reuptake inhibitors (SSRIs) and hematopoietic growth factors can be used as drug and particularly for treating hematopoietic diseases and/or cytopenia. The present invention in particularly suited for patients in need of chemotherapy and more particularly to reduce length of aplasia after chemotherapy.

### REFERENCE

L. B. et al. Comparison of haematological recovery times and supportive care requirements of autologous recovery phase peripheral blood stem cell transplants, autologous bone marrow transplants and allogeneic bone marrow transplants. Bone marrow transplantation 9, 277-284 (1992).
L. B., Dyson, P. G. & Juttner, C. A. Cell-dose effect in circulating stem-cell autografting. Lancet 2, 404-405 (1986).
J.P. Feighner, Mechanism of action of antidepressant medications, The Journal of clinical psychiatry 60 Suppl 4614 (1999) 4-11; discussion 12-3.
V. Erspamer, Experimental research on the biological significance of enterochromaffin cells, Arch. Fisiol. 37 (1937) 156-159;
V. Erspamer, B. Asero, Identification of enteramine, the specific hormone of the enterochromaffin cell system, as 5-hydroxytryptamine, Nature 169 (4306) (1952) 800-801
S.N. Spohn, G.M. Mawe, Non-conventional features of peripheral serotonin signalling - the gut and beyond, Nature reviews, Gastroenterol. Hepatol. 14 (7) (2017) 412-420
P. Amireault, D. Sibon, F. Cote, Life without peripheral serotonin: insights from tryptophan hydroxylase 1 knockout mice reveal the existence of paracrine/autocrine serotonergic networks, ACS Chem. Neurosci. 4 (1) (2013) 64-71.
Mosienko V, Beis D, Pasqualetti M, Waider J, Matthes S, Qadri F, Bader M, Alenina N.Behav, . Life without brain serotonin: reevaluation of serotonin function with mice deficient in brain serotonin synthesis. Brain Res. 2015 Jan 15;277:78-88.
Fouquet G, Coman T, Hermine O, Côté F. Serotonin, hematopoiesis and stem cells. Pharmacol Res. 2018 Aug 11. pii: S1043-6618(18)30482-1.

## Claims

1. A combination of selective serotonin reuptake inhibitors (SSRIs) and hematopoietic growth factors for use as drug.

2. Combination of selective serotonin reuptake inhibitors (SSRIs) and hematopoietic growth factors according to claim 1, wherein said drug is administered after chemotherapy.

3. Combination of selective serotonin reuptake inhibitors (SSRIs) and hematopoietic growth factors according to claim 2, wherein the chemotherapy is an aplasia chemotherapy.

4. A combination of selective serotonin reuptake inhibitors (SSRIs) and hematopoietic growth factors for use in the treatment of hematopoietic diseases.

5. Combination of selective serotonin reuptake inhibitors (SSRIs) and hematopoietic growth factors for use in the treatment of cytopenia.

6. Combination of selective serotonin reuptake inhibitors (SSRIs) and hematopoietic growth factors according to claim 4 or 5, wherein diseases involve hematopoietic stem and progenitor cells regeneration.

7. Combination of serotonin reuptake inhibitors (SSRIs) and hematopoietic growth factors according to claim 4 or 5, wherein said diseases involve hematopoietic stem cell transplantation (HSCT)

8. Combination of selective serotonin reuptake inhibitors (SSRIs) and hematopoietic growth according to claim 4, wherein hematopoietic diseases is hematopoietic malignancies

9. Combination of selective serotonin reuptake inhibitors (SSRIs) and hematopoietic growth according to claim 8, wherein hematopoietic malignancies is myelodysplastic syndrome.

10. Combination of selective serotonin reuptake inhibitors (SSRIs) and hematopoietic growth factors according to claims 1 to 9, wherein combination is used in allograft or autograft context.

11. Combination of selective serotonin reuptake inhibitors (SSRIs) and hematopoietic growth factors according to claim 10, wherein autograft context is a hematopoietic stem cells (HSCs)-autograft or allograft context is a hematopoietic stem cells (HSCs)-allograft

12. Combination of selective serotonin reuptake inhibitors (SSRIs) and hematopoietic growth factors according to claims 1 to 11, wherein drug is administered to improve Hematopoietic Stem and progenitor Cells mobilization and/or engraftment function.

13. Combination of selective serotonin reuptake inhibitors (SSRIs) and hematopoietic growth factors according to claim 12 wherein, Hematopoietic Stem Cells are collected in the peripheral blood after mobilization.

14. Combination of selective serotonin reuptake inhibitors (SSRIs) and hematopoietic growth factors according to claim 1 to 13, wherein serotonin reuptake inhibitors (SSRIs) is selected from the group consisting in fluoxetine, Citalopram, Sertraline, Paroxetine, Escitalopram, Fluvoxamine.

15. Combination of selective serotonin reuptake inhibitors (SSRIs) and hematopoietic growth factors according to claim 1 to 13, wherein hematopoietic growth factors is selected from the group consisting in Erythropoietin, Thrombopoietin, G-CSF, IL-3, 11-6, GM-CSF, G-CSF, PDGF.

16. Pharmaceutical kit comprising selective serotonin reuptake inhibitors (SSRIs) and hematopoietic growth factors for use as drug.
